# EUROPEAN PATENT SPECIFICATION

(11) **EP 1 593 076 B1**
(45) Date of publication and mention of the grant of the patent: **30.10.2019**
(21) Application number: 04701169.7
(22) Date of filing: 09.01.2004
(51) Int. Cl.: G16H 70/20

(54) **MEDICATION MANAGEMENT AND EVENT LOGGER ANALYSIS SYSTEM**
ARZNEIMITTELVERWALTUNGS- UND EREIGNISREGISTRIERUNGS- ANALYSESYSTEM
SYSTEME DE GESTION DES MEDICAMENTS ET DE CONSIGNATION D'EVENEMENTS

(30) Priority: 09.02.2003 US 361704
(43) Date of publication of application: 09.11.2005
(73) Proprietor: CareFusion 303, Inc., San Diego, CA 92130 (US)
(72) Inventor: VANDERVEEN, Timothy, W., Poway, CA 92064 (US); BATCH, Richard, M., Del Mar, CA 92014 (US)
(74) Representative: Richards, John
(86) International application number: PCT/US2004/000443
(87) International publication number: WO 2004/072828

(56) References cited:
- WO-A-01/88828
- WO-A-02/36044
- WO-A-02/069099
- WO-A-2004/061745
- WO-A1-99/62403
- US-A1- 2003 009 244
- ESKEW J A ET AL: "Using innovative technologies to set new safety standards for the infusion of intravenous medications", HOSPITAL PHARMACY, LIPPINCOTT, PHILADELPHIA, US, vol. 37, no. 11, 1 November 2002 (2002-11-01), pages 1179-1189, XP002344385, ISSN: 0018-5787
- Paul Govern: "IV pump prevents mistakes", Reporter - Vanderbilt University Medical Center's Weekly Newspaper, 15 March 2002 (2002-03-15), XP055484290, Retrieved from the Internet: URL:http://www.mc.vanderbilt.edu/reporter/ index.html?ID=1989 [retrieved on 2018-06-14]

## Description

### BACKGROUND OF THE INVENTION

The present invention relates generally to systems and methods for managing patient care in a health care facility, and more particularly, to systems and methods for integrating and managing information with respect to medical care, medication delivery, asset identification, and verification of drug delivery.

Medication errors, that is, errors that occur in the ordering, dispensing, and administration of medications, regardless of whether those errors caused injury or not, are a significant consideration in the delivery of healthcare in the institutional setting. Additionally, adverse drug events ("ADE"), which are a subset of medication errors, defined as injuries involving a drug that require medical intervention, and representing some of the most serious medication errors, are responsible for a number of patient injuries and death. Healthcare facilities continually search for ways to reduce the occurrence of medication errors. Various systems and methods are being developed at present to reduce the frequency of occurrence and severity of preventable adverse drug events ("PADE") and other medication errors. In the administration of medication, focus is typically directed to the following five "rights" or factors: the right patient, the right drug, the right route, the right amount, and the right time. Systems and methods seeking to reduce ADE's and PADE's should take these five rights into consideration.

Several companies are currently marketing or will be marketing systems that are designed to provide drug administration scheduling, drug administration verification, and the electronic documentation of drug administration. These devices are predominantly used to verify administration of oral, intramuscular ("IM"), subcutaneous, and topical drugs and have limited capability in verifying the administration of intravenous ("IV") drugs. One disadvantage of these devices is that they are currently incapable of monitoring or receiving data regarding the initial and ongoing infusion parameters of an IV infusion device. Moreover, even if the system is capable of monitoring the input of medication administration parameter values into a medication administration device, the system may not be capable of monitoring whether the entered value is within institutionally established guidelines, and if not, of providing an alert signal to the operator of the device.

When incorrect treatment parameter values are entered into a therapeutic device (medication administration device), the occurrence, for the purposes of describing the present invention, is defined as an "event." While some presently available medication administration devices are capable of storing information concerning an event, there currently exists no system which is capable of retrieving the stored "event" information, analyzing the information, and providing reports to the institution or caregiver. Presently, such information must be downloaded from a medication information device and manually analyzed by a skilled technician. US-A1-2003/0009244 discloses a care management system in which the management of the administration of care for patients is automated. Hospital information systems are monitored and the information from those systems is used in verifying the administrations of care to patients. The care management system monitors ongoing administrations for progress and automatically updates records and provides alarms when necessary.

It would be useful to have a system where such "event" information is automatically downloaded, either by command or as a matter of routine, for each medication administration device, or at least a selected subset of devices, that could be combined for analysis and reporting back to the institution in a predetermined format established by the institution. Such reports would be valuable in assisting the institution in monitoring the delivery of medication within the institution and to identify areas or procedures needing improvement to decrease the number or type of events being reported. Such reports would also be helpful in identifying medication administration device operators requiring additional or different training to ensure proper administration of medications within the institution.

It would be advantageous to have a care management system that combines all the various medication order and administration services of a healthcare facility into an integrated, automated system that checks and documents the delivery of therapeutic and other drugs to the patient. Such a system could help prevent administering an inappropriate medication to a patient by checking the medication and medication delivery parameters against a database of institutionally established medication administration guidelines. Additionally, the system may compare known allergic reactions and side-effects of the drug against the patient's medical history. The integrated system should also provide doctors, nurses, and other care-givers with updated patient information at the bedside, notify the facility's pharmacy when an additional drug is required, or when a scheduled treatment is running behind schedule, and automatically update the facility's accounting database each time a medication or other care is given.

In many hospitals and clinical laboratories, a bracelet device having the patient's identification, such as his or her name printed thereon, is affixed to a patient upon admittance to the facility in order to identify the patient during his or her entire stay. Despite this safeguard, opportunities arise for patient identification error. For example, when a blood sample is taken from a patient, the blood sample must be identified by manually transcribing the patient's name and other information from the patient's identification bracelet. In transferring the patient's name, a nurse or technician may, instead of actually reading the patient's bracelet, miscopy the name or may rely on memory or a different data source. Moreover, manually transferring other information such as parameters for configuring an infusion pump to dispense medication may result in errors that reduce the accuracy and/or effectiveness of drug administration and patient care. This may result in an increased duration of treatment with an attendant increase in cost.

Hospitals and other healthcare institutions continuously strive to provide quality patient care. The possibility of medical errors, such as where the wrong patient receives the wrong drug at the wrong time, in the wrong dosage, or even where the wrong surgery is performed, are a significant concern for all healthcare facilities. Many prescription drugs and injections are identified merely by slips of paper on which the patient's name and identification number have been handwritten by a nurse or technician who is to administer the treatment. For a variety of reasons, such as the transfer of patients to different beds and errors in marking the slips of paper, the possibility arises that a patient may be given an incorrect treatment. This could be prevented by using an automated system to verify that the patient is receiving the correct care. Various solutions to these problems have been proposed, such as systems that use bar codes to identify patients and medications, or systems allowing the bedside entry of patient data. While these systems have advanced the art significantly, even more comprehensive systems could prove to be of greater value.

Delivery, verification, and control of medication in an institutional setting have traditionally been areas where errors can occur. In a typical facility, a physician enters an order for a medication for a particular patient. This order may be handled either as a simple prescription slip, or it may be entered into an automated system, such as a physician order entry ("POE") system. The prescription slip or the electronic prescription from the POE system is routed to the pharmacy, where the order is filled, so that the medication can be provided to the patient. Typically, pharmacies check the physician order against possible allergies of the patient and for possible drug interactions in the case where two or more drugs are prescribed, and also check for contra-indications. Depending on the facility, the medication may be identified and gathered within the pharmacy and placed into a transport carrier for transport to a nurse station. Once at the nurse station, the prescriptions are again checked against the medications that have been identified for delivery to ensure that no errors have occurred.

Typically, medications are delivered to a nurse station in a drug cart or other carrier that allows a certain degree of security to prevent theft or other loss of medications. In one example, the drug cart or carrier is divided into a series of drawers or containers, each container holding the prescribed medication for a single patient. To access the medication, the nurse must enter the appropriate identification to unlock a drawer, door, or container. In other situations, inventories of commonly-used drugs may be placed in a secure cabinet located in an area at or close by a nurse station. This inventory may contain not only topical medications but oral, IM-, and IV-delivered medications as well. Nurse identification and a medication order number are typically required to gain access to the cabinet.

The nurse station receives a listing of drugs to be delivered to patients at intervals throughout the day. A nurse or other care-giver or other qualified person reads the list of medications to be delivered, and gathers those medications from the inventory at the nurse station. Once all of the medications have been gathered for the patients in the unit for which the nurse station is responsible, one or more nurses then take the medications to the individual patients and administer the dosages.

Common to all of these systems is the nurse who delivers the medication. The nurse is central to the process of verifying that the right medication is given to the right patient in the right dosage at the right time at the point of care. No other person in the facility is situated as well as the nurse delivering the medication to ensure or verify that the appropriate drug is being given to the appropriate patient.

Such a system works well to verify that patients are receiving the appropriate drug when drugs are delivered orally, but the system may not be capable of thoroughly verifying that the appropriate medication regimen is being delivered to a patient in the case where IV drugs are being delivered. For example, a nurse may carry an IV bag to a particular patient area, hang the bag, program an infusion pump with appropriate treatment parameters, and begin infusion of the medication. The applicable hospital control system, such as the pharmacy information system, may not know that the patient has received the medication, and if the information is lost somewhere, the possibility exists of medicating the patient twice. Thus, there may be a break in the link of verification that the medication is being properly delivered to the patient if an event occurs resulting in a deviation from the desired treatment parameters.

Moreover, even where the right medication arrives at the right patient for administration, incorrect administration of the medication may occur where the medication is to be administered using an automated or semi-automated administration device, such as an infusion pump, if the automated device is programmed with incorrect medication administration parameters. For example, even where the medication order includes the correct infusion parameters, those parameters may be incorrectly entered into an infusion pump, causing the infusion pump to administer the medication in a manner that may not result in the prescribed treatment.

One attempt at providing a system with built-in safeguards to prevent the incorrect entry of treatment parameters utilizes a customizable drug library which is capable of monitoring the parameter entry process and interacting with the care-giver should an incorrect entry or an out of range entry be attempted. In such a case, an alert is communicated to the care-giver that the parameter entered is either incorrect or out of a range established by the institution where care is being provided.

Additionally, various methods have been used to record all of the activities surrounding the delivery of a treatment regimen, such as providing an infusion pump with a memory dedicated to storing a record of events related to a particular treatment. For example, in one system, an infusion pump has a memory in which treatment information, including treatment parameters, patient identification, care-giver identification and other information are stored for later retrieval. Alternatively, the infusion pump may be programmed to store information related to only certain events occurring during treatment delivery, such as the occurrence of alarms or other alerts. Such systems typically require that the infusion pump be connected to a separate computer using an appropriate communication protocol to query the memory and download a copy of the stored events for analysis. Such information retrieval requires that each infusion pump be connected and analyzed separately, requiring large expenditures of skilled technician time. Additionally, the information retrieved from the memory is generally in a raw form that requires additional analysis before it is useful to an institution to determine if there are particular events or procedures that cause recurring problems and which require attention to improve the safety and efficiency of treatment delivery.

WO0188828 A2 provides a system and method for communicating and validating patient information including medication delivery information in a care-giving facility. A medical transaction carrier communicates information regarding medication delivery between a control system in communication with the care-giving facility's other information systems and a patient specific asset such as an infusion pump. All information carried by the medical transaction carrier is validated both at the patient specific asset and at the control system.

Hence what has been recognized as a need, and has heretofore been unavailable, is an integrated, modular system for tracking and controlling patient care and confirming that the correct medication administration parameters are entered into an automatic or semi-automatic medication administration device, and which also may be configured to store the medication administration parameters for later communication to, and integration with, other institutional databases to achieve accurate, reliable, efficient, and cost-effective delivery of health care to patients. What is also needed is an automated system for retrieving and analyzing information and events related to the delivery of medication. Such a system would also be capable of sending a report containing the analysis in a format predetermined or selected by the institution to personnel within the institution for use in improving the safety and efficiency of treatment delivery in the institution.

### SUMMARY OF THE INVENTION

Briefly, and in general terms, the present invention is directed to a method of reducing a risk of medication errors according to claim 1 and a system according to claim 2.

### BRIEF DESCRIPTION OF THE DRAWINGS

FIGURE 1 is a and graphical representation of a care management system incorporating principles of the present invention and illustrating details of the flow of information within the system;
FIG. 2 is functional block diagram illustrating the flow of information between medication administration devices and systems for controlling and monitoring medication administration within an institution in accordance with aspects of the present invention;
FIG. 3 is a functional block diagram illustrating one embodiment of a system architecture depicting the flow of information within a patient care system in accordance with aspects of the present invention;
FIG. 4 is a graphic representation of a patient identification bracelet including a barcode that can be read by a barcode reader;
FIG. 5 is a drawing of a barcode label affixed to a medication container that can be read by a barcode reader;
FIG. 5A is a drawing showing a barcode label affixed to a caregiver identity badge;
FIG. 6 is a drawing showing a sheet of barcode labels that can be affixed to various containers or devices;
FIG. 7 presents a view of a patient having an identification device located on his arm that interacts with a transmitter/receiver located in the frame of the entry/exit of the room in which the patient is located. The identification device and transmitter/receiver form a passive identification system in accordance with an aspect of the invention;
FIG. 8 is a graphical representation of another embodiment of the care management system showing the clinical devices connected to the local area network through a bedside data concentrator;
FIG. 9 is a graphical representation of still another embodiment of the care management system showing the clinical devices transmitting and receiving information from the local area network through RF transmitting/receiving equipment;
FIG. 10 is a graphical representation of another embodiment of the care management system of the present invention where all of the hardware elements of the local area network communicate with each other using RF transmitting/receiving equipment;
FIG. 11 is a graphical representation of another embodiment of the care management system of the present invention wherein a library or libraries of various patient treatment related information are stored in the memory of a medication database carrier which may be configured to communicate with an institutions systems using either a hard wired or wireless communication system.

### DETAILED DESCRIPTION OF THE PREFERRED EMBODIMENTS

The present invention provides a system and method of reducing a risk of medication errors. Additionally, the present invention also provides for verifying that the right treatment has been given to the right patient.

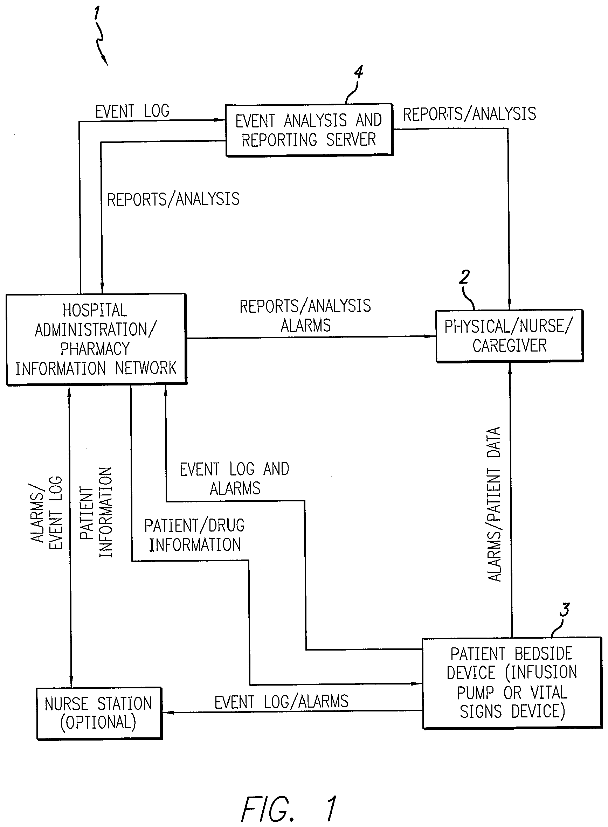

Referring now to the drawings in which like reference numerals are used to refer to like or corresponding elements among the several figures, there is generally shown in FIGURE 1 an integrated care management system 1 in accordance with aspects of the present invention. In accordance with the present invention, care management system 1 includes a communications network that ties together various sub-systems at the care giving institution as well as provides for communications to analysis and reporting systems that may be located on-site at the institution, but which may also be located off-site at a location different from the institution.

As depicted in FIG. 1, the system in accordance with the present invention provides for various databases, communication networks or systems for providing communication between the databases and various processors, microprocessors, computers, PDAs or other systems at the institution. As shown in FIG. 1, patient medication information and other non-medication related information may be entered and stored in a hospital administration system and/or a pharmacy information system. Information from these systems, particularly medication information, may be communicated to nurses and care-givers 2, and to the patient's bedside 3. Information generated or collected by various medication administration devices and/or vital signs monitoring devices may be communicated to the hospital administration system and/or pharmacy information system, and alarms or other information may be communicated from the patient's bedside 3 to the nurse or other care-giver 2.

After medication is administered, information collected from the patient's bedside by medication administration devices or vital signs monitoring devices may be communicated to an event analysis and reporting server 4. This information may be analyzed and reported either by patient, or the information from many patient's may be consolidated and analyzed and reported according. The reports may be generated on a custom basis, that is, individual reports may be requested, or reports may be generated in a format pre-established by the institution. The reports and analysis may be communicated back to the hospital administration and/or pharmacy information systems, or they may be communicated directly to physicians, nurses or care-givers, or any combination of departments or individuals within the institution that request the reports, or who might benefit from the information and analysis contained within the reports.

The communications systems connecting each of the hospital administration and pharmacy information systems, the event analysis and reporting server, the nurse or other care-giver, and the devices at the patient's bedside may be hard wired, wireless, or any combination of both hard wired and wireless elements.

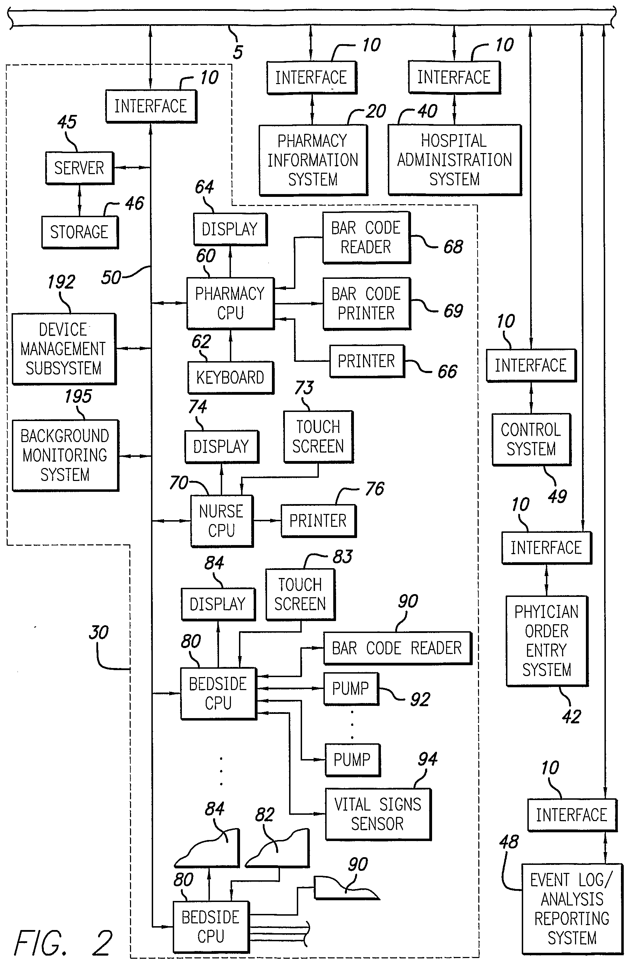

As shown in FIG 2, various subsystems of a facility's information management system are connected together by way of a communication system 50. The communication system 50 may be, for example, a local area network (LAN), a wide area network (WAN), Internet- or Intranet-based, or some other telecommunications network designed to carry signals allowing communications between the various information systems in the facility. For example, as shown in FIG. 2, the communication system 50 connects, through various interfaces 10, a hospital information system 40, a pharmacy information system 20, a physician order entry system 42, an event logging/analysis and reporting system 48, and a control system 49. The care management system embodiment shown in FIG. 2 is depicted as being configured as a local area network with a file server 45 to which are connected a pharmacy computer 60, a nursing station 70, and bedside CPUs 80. The file server 45 stores programs and data input and collected by the various computers in the local area network. Various application modules of the patient management system may be resident in each of the computers in the network and will be discussed in more detail below. Ethernet cabling of a local area network 50 is used to connect various CPUs to the file server. The file server 45 also has both local and network hard disk storage for storing programs as well as data gathered on the network. It will be understood by those skilled in the art that all of the ethernet cabling may be replaced using a wireless communication system, as will be described in more detail below.

Each of the various systems 20, 40, 42, 48 and 49 are typically interconnected via a network 50 and appropriate interfaces 10, and generally comprise a combination of hardware such as digital computers which may include one or more central processing units, high speed instruction and data storage, on-line mass storage of operating software and short term storage of data, off-line long-term storage of data, such as removable disk drive platters, CD ROMs, or magnetic tape, and a variety of communication ports for connecting to modems, local or wide area networks, such as the network 5, and printers for generating reports. Such systems may also include remote terminals including video displays and keyboards, touch screens, printers and interfaces to a variety of clinical devices. The processors or CPUs of the various systems are typically controlled by a computer program or programs for carrying out various aspects of the present invention, as will be discussed more fully below, and basic operational software, such as a Windows™ operating system, such as Windows NT™, or Windows 2000™, or Windows XP, distributed by Microsoft, Inc., or another operating program distributed, for example, by Linux, Red Hat, or any other suitable operating system. The operational software will also include various auxiliary programs enabling communications with other hardware or networks, data input and output and report generation and printing, among other functions. Further, while the control system 49 is shown as a separate system in FIG. 2, it will be understood that the control system 49 and the associated mass storage may also be incorporated into another element, such as an infusion pump or other system.

The communication system 50 may comprise, for example, an Ethernet (IEEE 522.3), a token ring network, or other suitable network topology, utilizing either wire or optical telecommunication cabling. In an alternative embodiment, the communication system 50 may comprise a wireless system, utilizing transmitters and receivers positioned throughout the care-giving facility and/or attached to various computers, clinical devices and other equipment used in the facility. In such a wireless system, the signals transmitted and received by the system could be radio frequency (RF), infrared (IR), or other means capable of carrying information in a wireless manner between devices having appropriate transmitters or receivers may be used. It will be immediately understood by those skilled in the art that such a system may be identical to the system set forth in FIG. 2, with the exception that no wires are required to interconnect the various aspects of the system.

In one embodiment of the present embodiment, the file server 45 of the care management system is connected by a local area network (LAN) 50 to computers and other peripheral equipment located in the institution's pharmacy, at nursing stations located throughout the institution, and at the patient's bedside. In the embodiment shown, the module located in the pharmacy comprises a central processing unit 60 to which is attached a video display 64 and a keyboard 62 for entry and display of patient information and drug parameters. Also attached to the pharmacy CPU is a bar code reader 68 which is adapted to read barcode labels that may be attached to drug containers, equipment, or caregiver identification badges as will be more fully discussed below. Also connected to the pharmacy CPU 60 is a bar code printer 69 and a printer 66 used for generating reports containing information about patient history and/or patient treatment. The printer 66 may also be used to print barcode labels generated by the pharmacy CPU 60 after patient or drug data is input by a technician or pharmacist into the pharmacy computer 60 using the keyboard 62 or other means.

Another computer, herein referred to as the nursing CPU 70, is located at a nursing station. Nursing stations are typically located in various sections and/or floors of a hospital or clinic and typically provide a central location for record storage and monitoring for a number of patient beds. The nursing CPU 70 located at the nurse station typically includes a video display 74 for displaying patient or other information pertaining to the operation of the particular unit of the institution, and a keyboard 72, mouse, touch screen 73, or other means for entering patient data or specific commands instructing the nursing CPU 70 to generate reports relating to either the patient's medical history or the course and progress of treatment for an individual patient on the attached printer 76 or on the video display 74. As will be discussed more fully below, the nursing station CPU 70 may also generate other reports such as, for example, a printout of drugs scheduled to be administered to patients, productivity measurements such as, for example, the amount of time a nurse spends with a patient or other reports useful for assisting in the efficient operation of the particular unit or the hospital. For example, a report listing the actual times of administration versus the scheduled times for administration may be prepared to assist in evaluation of staffing requirements.

Each care unit associated with the nursing station typically comprises one of more patient beds located in private rooms, shared rooms, or open or semi-open wards that contain multiple beds. In accordance with an embodiment of the present invention, each private room, semi-private room, or ward area has at least one bedside CPU 80 for monitoring and treating one or more patients. Each bedside CPU 80 has a video display 84 and a keyboard 82, mouse, touch screen 83, or other device. The bedside CPU 80 can be used by a nurse, physician, or technician to access a variety of institutional databases to display a variety of information about a particular patient. This information can include an on-line, real-time, graphical patient medication administration record (MAR) that is derived from the patient's medication profile maintained by the hospital's pharmacy information system 20. The bedside CPU 80 also allows remote access to a patient's records stored by the file server 45 to display medication history for the patient. This medication history includes a listing of all drug or other treatments including past, present and future deliveries to the patient. Additionally, access to administration records of the hospital's administration system 40 is available through the network 50. Alternatively, this information may also be stored, as will be discussed in more detail below, in a medication database carrier, the pharmacy information system, or a separate system dedicated to collecting, analyzing and producing reports concerning various alerts or clinical "events" that are recorded or logged during the administration of medical treatment to a patient.

In one embodiment of the present invention, a database including a library or libraries of information concerning past and present medical administration activities and/or institutional guidelines for appropriate parameters for administration of various medications. For example, the guidelines may include institutionally established guidelines or limits on drug administration parameters, such as dosage, frequency of administration, and other delivery related information such as, for example, appropriate flow rates and infusion durations for programming infusion pumps. Additionally, the guidelines may encompass guidelines for providing drug administration appropriate to a particular patient treatment areas having different sets of delivery parameters for similar medications, such as medication administration directed to geriatric, pediatric and oncology patients. Guidelines may also be included that are directed to particular therapy regimens, such as chemotherapy regimens or regimens for treating chronic infection or pain.

Each bedside CPU 80 can be connected through an appropriate interface to a variety of peripheral equipment. For example, a barcode reader 90 capable of reading barcodes on a patient's wristband or medication container; an infusion pump 92 for delivering medication to the patient in a predetermined, controlled manner; or various sensors 94 that can automatically monitor a patient's vital signs and send signals representative of these vital signs to the computer through an appropriate interface for storage and later retrieval by a selected software application to provide a graphic display of the patient's vital signs during the course of treatment.

A plurality of bedside CPUs are shown in the drawing; however, more or fewer may exist depending on the particular system and hospital requirements.

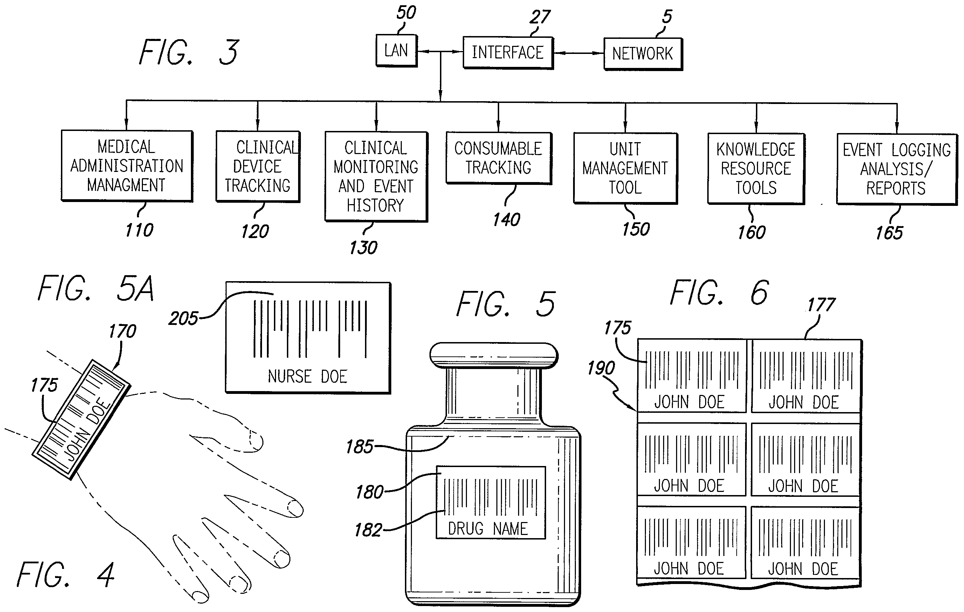

Referring now to FIG. 3, a block diagram illustrating the various application software modules comprising an illustrative embodiment of the care management system of the present invention is shown. The care management system's application software is modular in construction to allow installation and operation of the system with only one or more of the application software groups present. This provides flexibility in meeting the widely varying needs of individual institutions where cost and complexity may be an issue or where the full system is not needed. Each of the modular applications, however, is fully integratible into the system.

The programs of the care management system control alarms or alerts generated by one of the modular applications. Alarms are routed automatically to the appropriate video display. For example, an occlusion alarm generated by a pump 92 may remain local for a predetermined period. After that period the patient's bedside computer 80 may then broadcast the alarm by causing the alarm to be communicated over the LAN 50 to alert other hospital staff of a potential problem or to cause a particular person responsible for the care of a patient, such as, for example, a physician or nurse, to be paged.

Each of the modular applications will now be described in detail. The operation of each of these modular applications in a clinical setting will be discussed more fully below. The medical administration management module 110 integrates medical order information, infusion pump monitoring, and barcode technology to support the real-time verification and charting of medications being administered to a patient. The medical administration management module 110 creates and maintains an on-line, real-time, patient-specific medication administration record ("MAR") or integrated medication administration record ("IMAR") for each patient. This medication administration module 110 contains all of the information generated in the institution regarding the care provided to the patient. The medication administration management module 110 gathers information from the various nursing and bedside CPU's 70, 80 (FIG. 1) comprising the peripheral hardware of the care management system 30 that is distributed throughout the institution. For example, when a physician attending a patient diagnoses an illness and determines an appropriate course of treatment for the patient, the physician may prepare a handwritten medical order specifying the desired therapeutic treatment as well as any appropriate parameters such as dosage and/or period of administration. The written prescription is sent through the institutional mail system to the pharmacy where it is then entered into the pharmacy information system 20 through a dedicated terminal, or other means, and is then entered into the care management system.

In another embodiment, the physician accesses the pharmacy management system 20 through a dedicated terminal or through the care management system 30 via the network 5 using either a nursing CPU 70 or a bedside CPU 80. Alternatively, the treatment order may be entered by a nurse or other qualified caregiver into either the pharmacy management system 20 or the care management system 30.

Referring now to FIGS. 4-6, a variety of implementations of the barcode identification system of the present invention are shown. FIG. 4, for example, shows a patient identification bracelet 170 of the kind typically used in hospitals and other institutional settings to ensure that each patient is able to be identified even if the patient is unconscious or other-wise unable to respond to questioning. A barcode 175 is printed on a label that is attached to the patient identification bracelet 170 and has encoded within its sequence of bars the information necessary to identify the patient. This barcode may be read using a computerized barcode reader 68, 90, such as those shown connected to the pharmacy CPU 60 and the bedside CPUs 80 (FIG. 1). The barcode reader comprises a light emitting and receiving wand 95 that scans across the barcode. The light emitted by the wand 95 is reflected by the sequence of dark and light lines comprising the barcode into the receiving lens of the wand 95. A sensor in the wand 95 converts the received light into a signal that is then transmitted to the CPU. A software application program running on the CPU then decodes the signal into the data represented by the barcode in a manner well known to one skilled in the art. Using appropriate software programs, this data may then be automatically entered into a database stored in the CPU's memory or disk storage. While a barcode has been described for purposes of illustration, those skilled in the art will immediately understand that other systems, such as magnetic stripes, or programmed punched holes may also be used to represent data stored on each label, care giver badge or patient wrist band.

Barcode systems are extremely flexible and the amount of information that can be represented by the barcode, while limited, can be used in a variety of ways. For example, as depicted in FIG. 5, a drug container 185 is identified by a label 180 having a barcode 182 printed thereon. This barcode 182 can represent the patient identification and the medical order number, and any other information the institution finds helpful in dispensing the drug and tracking the treatment. The barcode 182 may also be read using a barcode reader, and, using suitable application software such as that included within the medical administration management module 110, discussed below, can be used to link the drug container and its contents with the patient identification bracelet 170 affixed to a patient to ensure the right drug is delivered to the right patient at the right time in the right manner. The use of barcodes is not limited to the implementations discussed above. A sheet 190 of barcode labels 177 having barcodes 175 is shown in FIG. 6. Such labels can be printed by a printer connected to the pharmacy CPU 60 of the care management system 30 or, alternatively, by any other printer connected to any other hospital information system that can be programmed to produce barcodes bearing the information in a form that can be read by the barcode readers connected to the various CPUs of the care management system. These barcode labels 177 may then be affixed to clinical devices, patient belongings, or other items where positive identification is needed. Alternatively, other devices may be affixed to the patient, drug, nurse or medical device that may communicate with the care management system using wireless means. For example, IR or RF transceivers may be incorporated into medication database carriers or other identification devices that are capable of interfacing and communicating with the care management system. Other wireless technologies may also be used.

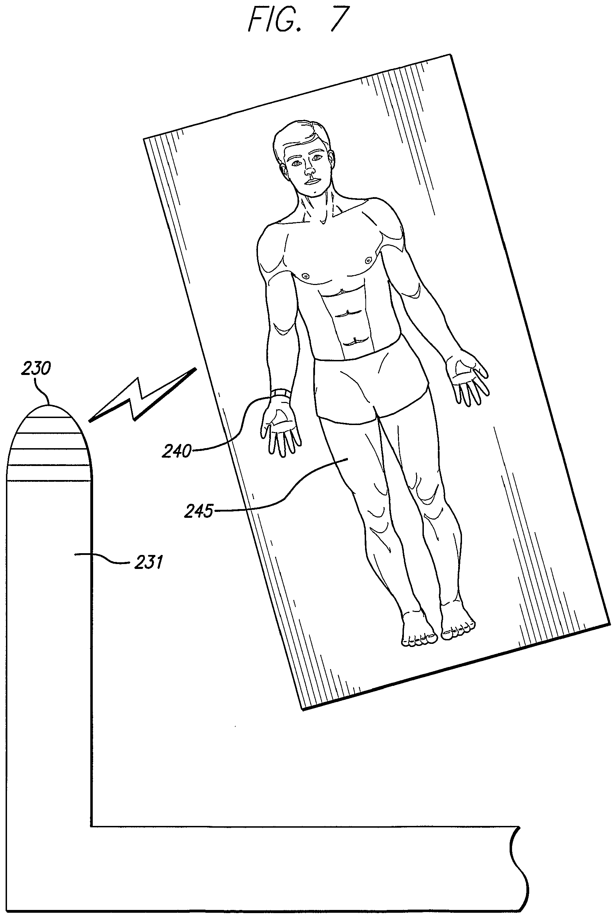

Another embodiment of the care management system is shown in FIG. 7 wherein the patient 245 and/or caregiver have badges or wrist bands 240 that may also include electronic circuitry that is responsive to signals from a transmitter/receiver 230 located in each patient room or treatment area to automatically provide the care management system (FIG. 2) with the identity of, and possibly other selected information about, the occupants of the patient room or treatment area, eliminating the need to use a bar-code reader to read the bar-codes on the patient and/or caregiver badges or wrist bands. Such a system may be described as a passive recognition system in that neither the patient nor the caregiver need take any active steps to inform the care management system of their location within the institution.

One example of such a system incorporates an intelligent RF computer chip into the caregiver or patient badge or wristband 240 that provides a unique, or programmed response with a passive RF transponder 230 located within a patient room or treatment area, such as in the frame 231 of the entry or exit of the room or treatment area, or mounted on a wall or ceiling. Each badge or wrist band 240 interacts with signals of the transponder 230 in a unique way, the unique interaction representing an assigned code for the badge or wristband 240. Utilizing this technology would remove manual steps and some of the "human factor" from the process of identifying the patient and caregiver.

When an individual 245 wearing a badge or wristband 240 having such a circuit enters a room or area where a transmitter/receiver 230 is located, the electronic circuit in the badge or wristband 240 interacts with signals emitted by the transmitter without any positive action on the part of the caregiver or the patient. This interaction may be sensed by the receiver, which may be capable of determining the identity of the badge or wristband 240 from the interaction of the electronic circuit with the emitted signals. Alternatively, the receiver may simply sense the interaction and provide a signal representative of the sensed interaction to a computer or other processor that has been programmed or otherwise configured to determine the identity of the individual associated with that particular badge or wristband 240.

Although the preceding paragraphs describe a passive recognition system using electrical circuitry, other approaches may also be used. For example, it can be envisioned that the patient and/or caregiver may have magnetically-encoded devices that can be automatically read by an appropriate detector located in the patient room or treatment area.

One of the key advantages of the medical administration management module 110 (FIG. 3) is that the module works in concert with the barcode labels or wire-less identification devices described above. When the medication administration management module 110 is implemented using the hardware system described above comprising a pharmacy CPU 60, barcode reader 68, and printer 66, together with a bedside CPU 80 with a connected barcode reader 90, the care management system ensures that medication is administered to the right patient, in the right dose, along the right route and at the right time.

Because the medication administration management module 110 maintains an on-line, real-time, patient specific graphical medication administration record that includes both past, present and future scheduled medications, a nurse may select a scheduled dosage on the MAR and indicate that it will not be administered for specified reasons selected from a list of options that are dependant upon the health status of the patient at a particular time. This system also allows a nurse to select a scheduled dose on the MAR, and record notes and observations about the dose selected from a list of options. The medical administration management module 110 also provides on-line, real-time help screens that can be accessed by a nurse or other caregiver to display specific information about selected medication and dose to be dispensed.

The medication administration management module 110 provides a list of on-going infusions that can be displayed on the video display of the pharmacy CPU 60. Drug administrations that will terminate within a preselected time period may be distinguished from other administrations by color highlighting or other means. The time remaining, drug, and patient name are presented as well as buttons for program control.

The medication administration module 110 records and maintains in a stored file a log of alerts that are generated when any discrepancy is identified, for example, during the verification process which will be discussed more fully below. The medication administration module 110 also allows the nurse to acknowledge and correct the discrepancy in real-time, or override the alert by entering the appropriate command. Even where the nurse is allowed to override the alert, the medication administration application module 110 prompts the nurse for a reason for each alert override and then automatically enters the reason into the MAR for the patient.

The medication administration module may also be capable of tracking specific alert conditions that are reported by specific medication administration devices indicating that particular treatment parameters have not been correctly entered into the device by a caregiver. These alerts, or "events" may be either automatically stored in a database associated with the medication administration module 110, or, as will be described more fully below, may be stored in a dedicated event logging/analysis and reporting server. The analysis may generate reports for a specified medication administration device or the analysis may consolidate event reports from all, or a selected subset of, the medication administration devices in an institution, and may provide reports in accordance with either customized formats or formats pre-established by the institution.

The clinical monitoring and event history module 130 shown in FIG. 3 is designed to monitor a variety of clinical devices attached to the network in a real-time manner and provides information about those devices to monitoring stations located elsewhere on the network. For example, the clinical monitoring and event history module 130 can be configured to monitor a plurality of clinical devices that are in use to deliver medication to patients in the private rooms, semi-private rooms or ward areas in a nursing unit. The clinical monitoring and event history module 130 retrieves real-time data from each device, and displays a visual representation of each device including all significant data related to its status and settings on the video display 74 connected to the Nursing CPU 70 (FIG. 2). For example, in the case where the clinical monitoring and event history module 130 is monitoring an infusion pump 92, a nurse at the nursing station can access the status for that pump wherein the display 74 attached to the nurse CPU 70 then displays information regarding the status of the infusion being performed at that time. For example, information can include the name of the drug being infused, the patient's name, the scheduled start, the actual start of infusion, the scheduled end of infusion, the projected end of infusion, the amount of drug infused, the amount of drug remaining to be infused and any alert or discrepancy conditions that may need attention by the nurse. Because the care management system is a fully integrated system, the medical administration management module 110 works in concert with the clinical monitoring and event history module 130 so that a nurse, doctor or technician may, after evaluating the status of the infusion displayed on either the video display 74 at the nursing CPU 70 or on the video display 84 at the bedside CPU 80 may, by using the touch screen 73, 83 of the computer, adjust the infusion regimen accordingly using, for example, a screen displayed on the video display 74, 84.

The clinical monitoring event history module 130 may also be programmed to immediately display alarm conditions on remote monitoring screens, such as the video display 74 attached to the nursing CPU 70, as the alarm occurs. For example, the status of each patient's infusion can be represented on a video display at the nursing station. When an alert occurs, the box representing the patient' room flashes red to attract attention to the alert. Displaying the alarm condition in this manner allows a nurse to quickly and easily identify the patient from the nursing station and take appropriate action to address the condition causing the alarm. The system may also be programmed to display certain alarms that have been identified as particularly important events at other video displays located throughout the institution, such as the video display 64 attached to the pharmacy CPU 60 located in the institution's pharmacy.

The clinical device tracking and reporting module 120 shown in FIG. 3 is used to maintain a record of the location of each clinical device and the history of its use in the institution. This system maintains a record of the current or last known location within the institution of each clinical device used in the institution, such as an infusion pump or vital sign sensor. Thus, the appropriate equipment can be easily located by a nurse or a technician for a given therapy regimen or vital sign measurement. This is particularly useful in a large hospital or clinic having many patient rooms, patient beds, or treatment areas where equipment may be temporarily misplaced. This system is also useful in those particular instances where an emergency occurs where treatment requires a particular piece of equipment. The status of that equipment can be easily ascertained from a remote video terminal, such as the video display 74 connected to the nursing CPU 70.

The clinical device tracking and reporting module 120 also maintains a record containing the usage history of each clinical device, including information about the patient it was used to treat, its location, the date, time, duration of use, any alarms that occurred and what medications were dispensed. This history may also contain the maintenance and calibration records for a clinical device. Such information can be queried on-line by technicians, nurses or other hospital administration personnel to generate reports to assist in locating the clinical device, report on the historical usage of the device, and to provide a log of preventative maintenance and equipment calibration. The efficient calibration of complex and sensitive clinical devices is particularly important in a heath care institution to maintain accuracy and quality of therapeutic treatment delivery. Maintaining a history of the usage of the device is also helpful to justify purchasing additional clinical devices when needed, or where the record indicates that a particular clinical device has become obsolete and needs to be replaced by a newer model of the device.

The care management system may also include a consumable tracking module 140 that maintains a record of all consumable item usage for treatment of each patient. This record ensures that appropriate supplies are ordered and delivered to the nursing unit in a timely and cost-efficient manner to prevent outages of necessary supplies. Such information may also be used by the hospital inventory systems through an appropriate interface or other management system to ensure that the supply purchasing is done as cost-effectively as possible. The consumable tracking module 140 provides on-line queries and report generation summarizing consumable uses for a particular patient, a particular nursing unit, or a variety of other purposes.

The unit management tool module 150 assists nurses in sharing information related to patients and automates routine transactions within the nursing unit. The unit management tool module 150 allows a nurse to record the allergies, handicaps, and special care needs of the patient which, cooperating with the medication administration record module 110 and the clinical monitoring and event history module 130, displays that information prominently on all appropriate display screens, either at the pharmacy video display 64, the nursing video display 74 or at the bedside video display 84 (FIG 2). The unit management tools module 150 also allows a nurse to record patient transfers and the times when the patient is out of the room or off the floor, such as, for example, when the patient is transferred to surgery or to a different part of the institution for a particular kind of treatment such as rehabilitative therapy. This system may also be programmed to signal an alarm when a patient has been disconnected from the system longer than scheduled, for example, when the patient disconnects from the infusion to attend to personal hygiene. This function ensures that an alarm or alert is sounded and that appropriate personnel are notified of any potential problems and can take the necessary actions to alleviate the alert condition.

The knowledge resource tools module 160 provides a framework for information sharing among the various units in the hospital and also supports an assortment of everyday tools used by the nurses, physicians and technicians involved in the delivery of health care within the institution. This module allows or assists in integrating external information sources into the care system to improve the effectiveness of the care management team in treating the patients in the institution.

For example, the knowledge resource tools module 160 may provide a variety of on-line tools including, for example, a calculator, a dose rate calculator for calculating the appropriate dosage and infusion rate for a particular drug to be infused into a patient, a standard measurement conversion calculator for converting between units of measurement, a skin surface area calculator, and a timer and stopwatch. These resources may be displayed on the video displays 64, 74, 84 at appropriate points within the system, and are available from any CPU either in the pharmacy, at the nursing station or at the bedside. These application tools can be programmed to appear on the video display 64, 74, 84 either automatically, such as, for example, when an infusion pump is configured at the start of an infusion to assist in the calculation of a dose rate. These resources may also be available upon entry of the appropriate command by a nurse, physician or technician.

One embodiment of the care management system of the present invention may include an event logging/analysis and reporting module 165, as depicted in FIG. 3. This module may be implemented in a variety of ways. For example, the event logging system 165 may be part of an institutions medication administration management module 110, it may be a separate module 165 as shown, or it may be implemented in a different computer system, which may or may not be located in the institution. For example, in one embodiment, event logging/analysis and reporting module 165 may resident on a third party computer system located outside of the institution, but in communication with the institution's systems using a wired or wireless, or combination of both, communication system.

A common feature of the various configurations of the event logging/analysis and reporting module 165 is that the module receives, or retrieves, information from medication administration devices related to alarms or alerts generated by the medication administration device before or during administration of medical treatments to a patient, analyzes the information, and then provides reports related to the received or retrieved information to the institution. These reports may be used by the institution to improve the delivery of medication to patients in the institution, by identifying frequently occurring errors or conditions that can be corrected through improvements to the medication delivery process or training or caregivers. Such reports may either be customized on demand, that is a caregiver or other individual responsible for analyzing the events may request a custom report, or a the system may provide a menu of reporting formats pre-established by the institution that may be selected by the individual or department requesting the report. Alternatively, the system may be automated so that reports in pre-established formats are produced and distributed to appropriate individuals or departments in the institution at pre-selected intervals. Such a system will typically be embodied in one or more databases stored in a memory from which event related information may be extracted and analyzed using a processor controlled by an appropriate software program. The results of the analysis may be stored in a memory for future use or distribution, or may be printed using a printer.

As depicted in FIG. 2, the care management system is connected to other systems in the institution via an interface 10. This interface may support standard health level 7 (HL7) interfaces to the hospital's other information systems and can also support custom interfaces to systems or devices that do not support the HL7 standard. The system interfaces may be either real-time or batch mode, although a real-time interface to a hospital's pharmacy system may be required to support the on-line medical administration records keeping function of the medical administration management module 110.

The care management system software can be written to operate on a variety of operating systems to suit the needs of a variety of institutions. In a present embodiment, the software is written to interface with the nurses and physicians using the Windows environment (Windows is a trademark of Microsoft, Inc.) on IBM compatible micro-computers. The Windows environment is well known by those skilled in the art and will not be described in detail herein. The care management system software, when implemented using the Windows system, is particularly useful in that the Windows operating system provides the ability to load several programs at once. Multitasking programs, allowing several application programs to run simultaneously yet providing immediate access to the various software modules of the care management system may also be used.

One particular mode of operation of the care management system will now be described. As described above, a patient entering a hospital or other care-giving institution is provided with a wristband necklace, ankle band or other identifier that is affixed to the patient in a manner so that the patient can be identified even if the patient is unconscious or otherwise unresponsive. Such a wristband 170 is depicted in FIG. 4. In one embodiment, the wristband 170 barcode represents the name of the patient and other information that the institute has determined is important and also includes a barcode 175. The information printed upon the band, such as name, age, allergies or other vital information is encoded into the barcode 175. It will be understood, particularly in view of the description above, that the barcodes and wristbands may be replaced with devices capable of communicating with the various institution systems using devices capable of wireless communication with the institution's systems.

After the patient is admitted and situated in a bed within the institution, the patient is typically evaluated by a physician and a course of treatment is prescribed. The physician prescribes the course of treatment by preparing an order, which may request a series of laboratory tests or administration of a particular medication to the patient. The physician typically prepares the order by filling in a form or writing the order on a slip of paper to be entered into the hospital's system for providing care.

If the order is for administration of a particular medication regimen, the order will be transmitted to the institution's pharmacy. The order will arrive in written form at the pharmacy, will be evaluated by the pharmacy, and processed. The pharmacy then prepares the medication according to the requirements of the physician. The pharmacy packages the medication in a container, such as the container 185 shown in FIG. 5. Normally, a copy of the order, or at a minimum, the patient's name, the drug name, and the appropriate treatment parameters are represented on a label that is then affixed to the drug container 185. According to one embodiment of the present invention, this information is represented by a barcode 182 that is then printed on a label 180. This barcode label 182 may be automatically generated using a printer capable of printing barcodes, such as, for example, a printer 69 attached to the hospital's pharmacy information system 20. The existence of this medication order is made available by the hospital's pharmacy information system 20 and is stored by the file server 45.

Generally, the medication is then delivered to the appropriate caregiving unit for administering to the patient. A nurse or technician carries the drug container 185 to the appropriate patient. In accordance with one embodiment of the present invention, the nurse or technician first read the barcode 175 on the patient ID bracelet 170 using the barcode reader 90 connected to the bedside CPU 80. The nurse or technician would then read the barcode 182 on the label 180 affixed to the drug container by swiping the barcode wand 95 across the barcode 182 printed on the label 180 of the drug container 185. Additionally, a record of the identity of the caregiver dispensing the medication may be obtained by reading the barcode 205 printed on an identity badge 200 (FIG. 5A) typically worn by all institution personnel.

For certain drugs, the care-giver is prompted to enter data descriptive of a selected patient parameter or parameters, such a laboratory value or a current vital sign, before completing the verification process. For example, the care-giver may be prompted to measure and enter a value for a patient's blood pressure before administering certain selected drugs. The system may include ranges of acceptable values for the parameters. If the system detects an out-of-range value for the parameter, the system causes an alarm to be provided. In an alternative embodiment, the parameters could be monitored and entered into the system automatically, eliminating the need for manual entry by the care-giver.

The data obtained then is analyzed by the medication administration management module 110 which records the therapeutic regimen information in the patient's MAR, and verifies that the right medication is being given to the right patient in the right dose by the right route and at the right time. If the medication administration management module 110 detects a discrepancy between the barcoded information printed on the patient bracelet 170 and the barcoded information on the label 180 affixed to the medication container 185, an alert is sounded and the appropriate information is displayed on the video display 84 attached to the bedside CPU 80. The nurse or technician then either corrects the discrepancy by either re-reading the barcode 175 on the patient's bracelet 170 and the barcode 182 on the medication container 185 or, alternatively, by entering the appropriate information into the bedside CPU 80 using the keyboard 82 or touch screen 83, mouse, or other device. In the event that the nurse or technician determines that the discrepancy cannot be automatically corrected by re-reading the barcodes and that the discrepancy is minor and will not affect the accuracy or safety of the delivery of the medication, the nurse or technician may override the alert.

In an embodiment of the present invention, where the medication is to be delivered using an infusion pump, such as the infusion pumps 92 attached to the bedside CPU 80, the care management system automatically downloads information consisting of the appropriate configuration parameters for the infusion from the pharmacy CPU 60 through the local area network 50 into the bedside CPU 80 and then into the infusion pump 92 when the verification function of the medical administration management module 110 is complete. This is particularly advantageous in that one potential source of inaccuracy is eliminated by automatically configuring the pump, thus eliminating the need for the nurse or technician to manually enter the parameters necessary to configure the infusion pump 92. In one embodiment, the infusion pumps 92 comprise IVAC Corporation Model 570 volumetric pumps. In an embodiment where the pumps cannot be automatically configured by downloading parameters from the network, the care management system 30 only verifies that the right treatment is being administered to the right patient. The pump must then be manually configured by the physician, nurse, or technician.

Alternatively, the nurse or caregiver may enter values for various treatment parameters into the pump manually. In this embodiment, the pump, or other medication administration device, may have incorporated within a memory associated with the pump or medication device, a library or libraries of information such as institutional guidelines for appropriate parameters for administration for various medications. For example, the guidelines may include institutionally established guidelines or limits on drug administration parameters, such as dosage, frequency of administration, and other delivery related information such as, for example, appropriate flow rates and infusion durations for programming infusion pumps. Additionally, the guidelines may encompass guidelines for providing drug administration appropriate to a particular patient treatment areas having different sets of delivery parameters for similar medications, such as medication administration directed to geriatric, pediatric and oncology patients. Guidelines may also be included that are directed to particular therapy regimens, such as chemotherapy regimens or regimens for treating chronic infection or pain.

In the present invention, a stored data base or library may contain preestablished "hard" and "soft" limit values on physiological parameters (such as CO2, SpO2, respiration rate, and others), PCA dosing parameters, and other infusion and vital sign parameters that have been established by the hospital or institution within which a patient care system in accordance with the present invention resides. Once medication administration values have been entered into the patient care system or medication administration device by a nurse or other care-giver, the processor of the medication device is programmed to compare each of these selected values against the stored library to verify that the selected values are within acceptable ranges. If a selected value contravenes a hard limit, the processor will alarm and require a value change before operation of the medication administration device can begin. If the selected value contravenes a soft limit, the processor of the medication administration device will require an acknowledgment from the nurse or other care-giver that he or she understands the value entered is outside a soft limit and that this value is nevertheless to remain in force. The library or libraries may not necessarily be located in the medication administration system but may be located elsewhere. For example, in the case where patient care systems or medication administration devices are connected to a hospital server, such a library may be located at the hospital server and the patient care system or medication administration device would communicate with the server during the verification stage to obtain the acceptable ranges. In another embodiment, the library may be located in a portable data assistant (herein "PDA") such as a Palm Pilot™ with which the patient care system or medication administration device may communicate via infrared link, RF, blue tooth, or by other means. The nurse or care-giver may carry the PDA and before the patient care system or medication administration device will begin operation, it must communicate with the PDA to compare the hard and soft limits against the entered values. Other library arrangements are possible.

Storing a data base of institutional standards for drug infusion parameters and physiological parameter limits, such as the maximum and minimum concentrations of CO2 and SPO2 and the maximum and minimum values of respiration rate, also aids in standardizing the quality of care in a clinical setting. In some embodiments, infusion parameter values or physiological parameter limits may be entered automatically from a machine-readable label, for example using a bar code reader mounted on the bag or on the syringe or other medical fluid container in which the medical fluid to be infused is stored. In other embodiments, such infusion parameter values and physiological parameter values may also be entered by other means, such as through a connection with an external processor, such as a hospital server, through connection to a PDA, or other. Connections with these devices may be made in various ways, such as direct, hardwired connection, infrared link, blue tooth link, or others.

The medical database system of one embodiment of the present invention receives medication administration information from a nurse or care-giver prior to medication administration, compares that information to institutionally established guidelines for administration of various medications, and provides an alert if any or all of the medication administration information received from the medication administration device falls outside of the guidelines stored within the medical database. This allows the nurse or care-giver administering the medication to correct the administration parameters entered into the medication administration device before medication administration to the patient is begun. If the administration information falls within the guidelines, the nurse or care-giver may receive a message that medication administration may begin. In one embodiment, the medication administration device may be "locked out", that is, electronically prevented from beginning administration of the medication until the medication administration device receives a signal from the processor that the administration parameters entered into the administration device are appropriate for the medication and that institutional guidelines for the administration have been met, unlocking the medication administration device and allowing the care-giver to begin medication administration.

In another embodiment, a separate library or libraries may be stored, either in the medication administration device or at another location that contains records of the medication administration parameters and/or events. The information stored in the library or libraries may be communicated to and incorporated with information in other institutional information systems, such as a pharmacy information system, or hospital information system, event logging, analysis and reporting system, or physician order entry system, or a patient specific asset located at a patient's bedside. The information stored in the library or libraries is used to validate that the right medication and the parameters of the medication administration record are properly delivered to the right patient. Additionally, in some embodiments, the information stored in the library or libraries may be analyzed and provided in a pre-established report format to the institution or care-giver to identify patterns and frequency of occurrence of logged events. In an alternative embodiment, the information stored in the library or libraries in communication with more than one medication administration device may be consolidated and analyzed, providing reports concerning the occurrence of events associated with selected areas within the institution, selected treatment protocols, or other categories as identified by the institution to assist the institution in ensuring the proper delivery of medication to patients within the institution.

A medical database in accordance with one aspect of the present invention may be a included in a device having a processor and a memory for storing information or databases, such as a personal data assistant ("PDA"), a laptop computer, a desktop computer, a smart card, a BLUETOOTH transceiver having a processor and memory, or other device capable of communicating with medication administration devices and storing and processing information. Such a medical data base carrier ("MDC") may either be portable, in the sense that the MDC may be moved about the institution, or the medical database carrier may be primarily stationary and located at the patient's bedside. At the patient's bedside, the medical database carrier is interfaced to a patient specific asset ("PSA"), such as an infusion pump or vital signs monitor.

Once the infusion pump or other medication administration device is configured, the nurse, caregiver, or technician starts the infusion by pressing the appropriate control on the infusion pump 92. Starting a pump that is capable of being monitored automatically by the care management system causes a signal to be transmitted from the pump to the bedside CPU 80 which is then logged by the clinical monitoring and event history module 130 and entered by the medical administration management module 110 into the patient's MAR. In the case where the institution is using a pump that is not capable of being configured by downloading parameters from the network, the nurse or other caregiver logs the start of the infusion using the touch screen device, mouse or other device connected to the bedside CPU 80. In this case, the video displays of the care management system that display information about the status of the infusion will not display real-time data. Rather, the care management system will project what the status of the infusion should be given the infusion parameters, the time elapsed since the infusion began, and any other events that were manually logged by the caregiver that may have affected the progress of the infusion.

The care management system, utilizing the application modules described above, monitors the infusion process in a real-time manner, providing alerts on the appropriate video display screens located throughout the institution and allows intervention by nurses or other caregivers at remote locations if necessary. If the pharmacy management system 20 is directly linked to the care management system, the care management system may also provide a scheduling report to the pharmacy in determining the status of ongoing infusions, as well as in scheduling the preparing of medications for future infusions.

In another embodiment, the present invention includes a "Code Mode" that allows a care-giver to bypass the system to immediately cause a list of drugs that have been preselected by the institution to be used in an emergency situation. The initiation of the "Code Mode" causes a time-stamp to be placed in the patient's MAR along with the identity of the drug selected from the displayed list of drugs to be used to treat the emergency. This feature ensures that the emergency and the treatment used to address the emergency are accurately recorded in the patient's MAR.

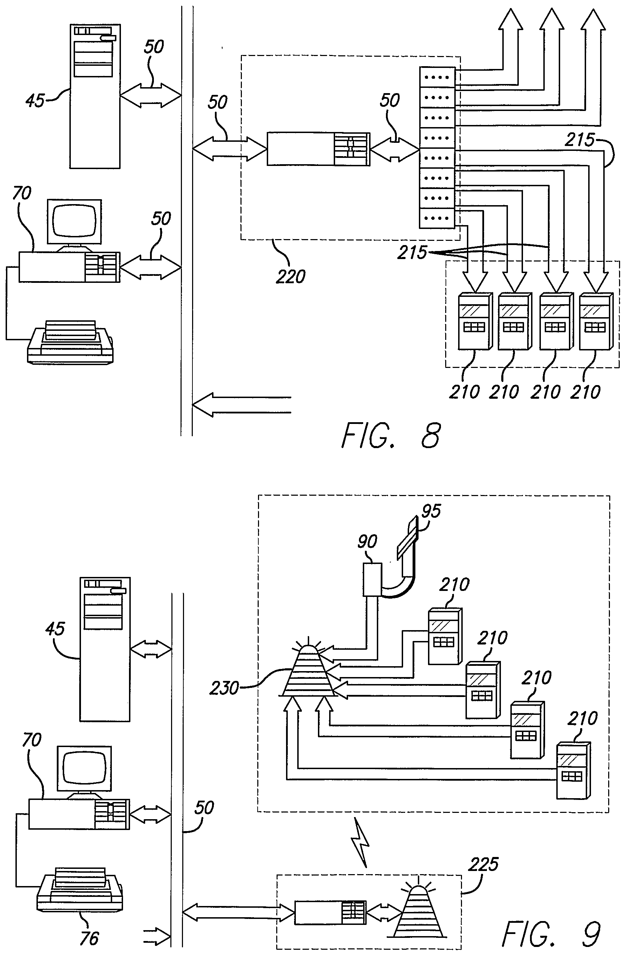

While one particular embodiment of the present invention has been described above, alternative configurations of the care management system network are possible. For example, one alternative embodiment of the care management system is depicted in FIG. 8. In this configuration, clinical devices 210 are connected by means of appropriate interfaces and cabling 215 to a bedside data concentrator 220 which would typically be located outside of a private room, semi-private room or ward area. In this configuration, there is no bedside CPU 80 as described previously. Instead, the bedside data concentrator 220 is connected through an appropriate interface and cabling to the local area network 50, where the data gathered from the clinical devices 210 is then available for processing by the care management system and display at the various monitoring stations, such as either in the pharmacy or at the nurse station 70. In this embodiment, there is no bedside CPU 80 having a keyboard 82 for data entry or a video display 84 for display of either clinical device information or patient information. As described previously, the devices may also communicate with each other and the communication system 50 by wireless means.

A further embodiment of the care management system local area network is depicted in FIG. 9. In this embodiment, the file server and monitoring stations are connected using appropriate interfaces and ethernet cabling to an RF data concentrator 225. At the bedside locations in the private rooms, semi-private rooms or ward areas of the institution, the clinical devices 210 and barcode reader 90 at the bedside are connected to an RF transmitter/receiver 230. This RF transmitter/receiver 230 transmits the information gathered from the clinical devices 210 and the barcode reader 90 to the RF data concentrator 225 attached to the local area network 50. Thus, expensive cabling is not required to connect every patient treatment area. Additionally, flexibility in locating the clinical devices 210 and barcode reader 90 is obtained as well as allowing the ability to reconfigure the patient treatment area without costly rewiring of the ethernet cabling.

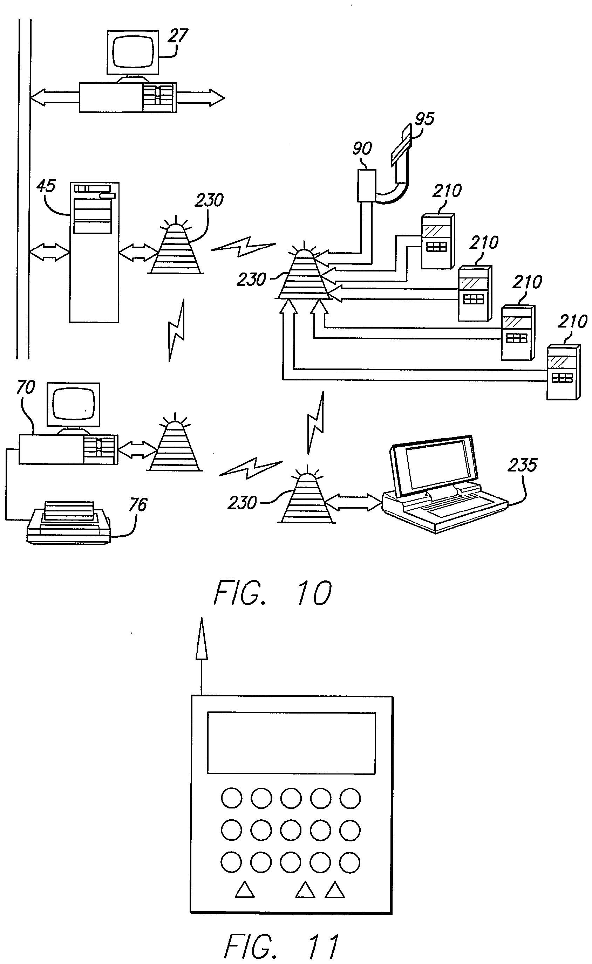

Yet another embodiment of the care management system local area network 50 configuration is shown in FIG. 10. In this configuration, the ethernet cabling connecting the pharmacy CPU, the nurse station nursing CPU 70 and bedside CPUs and clinical devices is eliminated entirely. Each hardware element, comprising the file server, nursing CPU 70, pharmacy CPU 60 and bedside CPUs 80 and clinical devices and/or barcode readers is connected to an RF transmitter/receiver 230. In this manner, all of the information is transmitted throughout the local area network 50 by way of radio transmission rather than by using costly network cabling. Such a system would additionally allow for the use of portable computers 235, PDAs, smart cards and other devices, such as portable medication data carriers, described more fully below, having RF transmitter/receivers 230 or other means of wireless communication, as have been described above, that could then be carried with physicians, nurses or technicians as they circulate through the institution. With this configuration, caregiving personnel could access the care management system either spontaneously or upon notification of an alert no matter where they were in the institution at any given time. Such a system would be particularly useful in a large institution where caregiving personnel are likely to be responsible for many hospital beds or when personnel are out of the area or off the floor. In accordance with aspects of the present invention, a medication database carrier ("MDC") 30, one embodiment of which is depicted in FIG. 11, including a processor and a memory for storing information is provided to monitor medication parameters or other information used by a nurse or other care-giver to program a medication administration device to deliver medication to a patient. Various types of information may be stored in the memory of the MDC 30, including databases containing information about drug interactions and possible contraindications and/or side-effects of medications, and a library or libraries of established guidelines for the administration of various medications. For example, the guidelines may include institutionally-established guidelines or limits on drug administration parameters, such as dosage, frequency of administration, and other delivery related information such as, for example, appropriate flow rates and infusion durations for programming infusion pumps. Additionally, the guidelines may encompass guidelines for providing drug administration appropriate to a particular patient or to treatment areas having different sets of delivery parameters for similar medications, such as medication administration directed to geriatric, pediatric, and oncology patients. Guidelines may also be included that are directed to particular therapy regimens, such as chemotherapy regimens or regimens for treating chronic infection or pain. The term "database" or "data base" as used herein will be understood by those skilled in the art to be used as is commonly understood, that is, the term "data base" refers to a collection of values or information organized, formatted, and stored in such a manner as to be capable of being retrieved and analyzed using an appropriate program contained in software or other form.

In one embodiment of the present invention, the MDC 30 may be interfaced to the nurse station computer system 70 (FIG. 2) or any other of the information systems of the central system of an institution through a cradle or other docking device that provides a connection between the MDC 30 and the care management system. This information may then be processed and stored on the care management system, or the information may be communicated by the care management system to various other institutional information systems over the communication system 50. In this manner, information from the pharmacy information system 20, for example, may be communicated through the communication system 50, the nurse station computer system 70, and the MDC cradle into the MDC 30. Similarly, information contained within the MDC 30 may be communicated through the MDC cradle, the nurse station computer system 70, and the communication system 50 to any of the interconnected systems 20, 40, 42, 48 and 49. Alternatively, the MDC may be capable of wireless communication with any or all of the interconnected systems 20, 40, 42, 48 and 49, or any other institutional system.

The medical database carrier 300 generally refers to a device that contains medication and/or patient specific information and/or databases or libraries, including institutionally generated guidelines for the delivery of medication to a patient, as well as drug interaction information or information concerning possible drug side-effects, and that is portable such that it can be carried by a nurse or other care-giver to and from a patient's bedside. Alternatively, as will be described in more detail below, the MDC 30 may be considered to be relatively stationary in that it is associated with either a particular patient or medication administration device. The MDC 30 may also have a storage capability and technology for interfacing with a computer system or network so that information may be communicated between the MDC 30 and other devices, such as computers, medication administration devices, clinical devices such as vital signs monitoring devices and the like. The MDC may also have a video display screen in color or black and white (mono-color), such as that provided by an LCD or an array of LED's, or other, and a data entry means such as a keyboard, keypad, a screen used for handwriting recognition, or other data entry means.

A general concept embodied in the MDC 30 is to provide a system and method wherein medication administration parameters or other information entered into a medication administration device such as an infusion pump, may be retrieved from the device prior to actual medication administration and compared to information in the database or databases stored in the MDC to determine if the entered parameters or information fall within institutionally established guidelines for the administration of a particular medication. If the comparison indicates that the parameters or information entered into the medication administration device are appropriate in that they fall within the established guidelines, then an indication to that effect is provided to the nurse or care-giver and the nurse may then begin medication administration.

Alternatively, if the comparison indicates that one or more parameters or information do not meet the established guidelines, a warning or alert is provided to the nurse or care-giver that one or more parameters or a portion of information has been incorrectly entered into the medication administration device, and that corrective action or an override is required before medication administration can begin. In another embodiment, the medication administration device may be automatically inhibited from starting administration of a medication unless it receives a signal from the MDC 30 that the comparison was favorable, thus providing a fail-safe against incorrect administration of the medication.

The MDC 30 typically will also be capable of retrieving medication administration parameters or information from a medication administration device, and storing data or information concerning various transactions in its memory representing the identity and treatment regimens for medications given to a patient, as well as other information, such as care-giver identity, equipment location, patient vital signs information, or any other information sought to be recorded. The MDC 30 may also store data or information concerning primary or secondary validation of previous and/or duplicate transactions of medical treatment information. The display of the MDC may also provide a care-giver with messages or other information, such as warnings or prompts to enter data, related to medication administration. Moreover, the keyboard or other information entry means of the MDC may be used for manually entering information into the MDC for storage in the memory of the MDC.

While specific examples of an MDC 60 are set forth herein, it will be understood that the MDC is meant to include any device that carries out the basic concept of the invention. That is, a device that receives medication administration or treatment information from a medication administration device, such as, for example, but not limited to, an infusion pump, and has a processor capable of comparing the received information to institutionally established medication administration guidelines or other pertinent information or data, such as drug interaction information and/or a library of possible side-effects, and then providing an indication of the result of the comparison to a nurse or care-giver before administration of a medication to a patient is begun, will accomplish the aims of the present invention. A particularly advantageous embodiment includes storing information about the medication administration, such as the medication administration or treatment parameters, and/or other information, such as the identity of the patient and care-giver, in the memory of the MDC 30 until the MDC 30 re-establishes a communication connection with the care management system, whereby the information stored in the memory of the MDC 30 may be communicated to the care management system and incorporated into one or more of an institution's information databases. Updating the databases provides a verification that the treatment has been rendered thereby avoiding a duplicate treatment. In this manner, the present invention "closes the loop" ensuring that the right medication has been given in the right manner to the right patient.

For example, consistent with the present invention, the MDC 30 may be embodied in a hand-held "personal digital assistant" ("PDA") such as a PalmTM Pilot or any PDA running either the PalmTm operating system or the Windows™ operating system, a desktop computer, a notebook computer, or other portable computer system. The MDC may also comprise a smartcard such as those that are capable of processing and storing data, such as the American Express Bluecard. The use of such devices is advantageous in that devices having a suitably large memory to accommodate the type of information required by the present invention to monitor and track medication administration information and validate treatment as well as retrieving other patient information, are readily available and relatively inexpensive, thus allowing an MDC to be assigned to each individual patient, or alternatively, to an individual medication administration device, such as an infusion pump, or other clinical device, such as a vital signs monitor. Additionally, such devices are small, compact and easily transportable.

Alternatively, the MDC 30 may be embodied in any device that includes an active embedded processor and a memory capable of storing information. Such an active embedded processor may be even smaller and more portable than a PDA or notebook computer. For the purposes of the present invention, such an active embedded processor includes any device incorporating a microprocessor and allows for input and/or output of information, whether via electrical, radio frequency, or optical means, wireless or direct contact, and which contains its own power supply. One example of an active embedded processor in accordance with this invention may be attached to or embedded in the packing or container of a medication to be delivered to a patient. Such devices may typically be manufactured no larger than, for example, a postage stamp or business card and yet include, using micro circuitry, enough processing power, information storage, data or information input and output, and power to be suitable for use as a medical database carrier. Alternatively, the embedded processor and memory may be integrated into a medication administration device, such as an infusion pump or other device.

In another embodiment, such as where the patient specific asset or medication administration device is modular and includes an advanced programming module ("APM"), such as in the ALARIS Medical Systems, Inc. MEDLEY™ MEDICATION SAFETY SYSTEM, the APM may include sufficient programming to perform the function of an MDC. In such case, the APM would be in contact with institutional information systems, such as the pharmacy information system 20, and receive updated information concerning institutional guidelines for medication administration or other patient area or drug specific information to be used to compare with entered medication administration information or parameters before beginning administration of a medication to a patient.

It is not unusual at present to find patient stations having a computer 80 (FIG. 2) located at patient bedsides in a care-giving facility. Such stations 80 may serve a single patient, or may serve more than one patient, depending on the design and arrangement of the patient area. There may also be a variety of equipment or clinical devices attached to the bedside computer 80. Examples of such devices are a bar code reader 90, a printer (not shown), patient monitoring equipment 94 for monitoring patient vital signs, or other patient specific assets assigned to the patient. Further examples of such PSA's include an infusion device 92 such as can form a part of the ALARIS Medical Systems, Inc.'s MEDLEY™ MEDICATION SAFETY SYSTEM 80. Attention is directed to U.S. Patent No. 5,713,856 entitled "Modular Patient Care System" to Eggers et al. in which the APM is described as an advanced interface unit. In such system, an infusion device may be mounted to an Advanced Programming Module. Other devices, such as a vital signs monitor or monitors, are envisioned as being mountable to the APM also. Other infusion or drug delivery devices and/or patient monitoring equipment such as cardiac or respiratory monitors may also comprise or form a part of the PSA.

The bedside equipment and clinical devices are typically equipped with data communication technology such as RS 232 serial ports or proprietary communication ports that allow information and data to be communicated to and from the equipment or clinical device. Using this communication technology, the bedside equipment and clinical devices may be connected to the bedside computer 80, or, alternatively, they may be connected, either by wire or wireless system, to the facility communication system 30 using wireless technology, such as RF, IR, or other wireless communication protocols.

As described previously, one particularly advantageous embodiment of the present invention includes an MDC 30 (FIG. 11) that is capable of communicating information to and from the a medication administration device and the institution' communication network 50 using wireless technology. For example, the MDC 30 may be understood to include, but is not limited to, communications utilizing optical or infrared transmission, magnetic transmission, or wireless technology where the wireless technology is understood to include methodology such as the BLUETOOTH™ technology (IEEE 522.15), standard methodologies such as wireless Internet, WAP or any other proprietary communication scheme using electromagnetic waves instead of wires to connect and communicate between devices. Such wireless communications may also be performed using other wireless networking alternatives, such as those described in the IEEE 522.11x standards. Wireless technologies are designed to create wireless networks allowing devices such as PDA's, cell phones, and personal computers to exchange information at relatively high transmission speeds.

Using BLUE TOOTH™ technology, for example, data from a medication administration device such as an infusion pump may be sent by an internal BLUE TOOTH™ communication device taking the form of a radio chip embedded in the medication administration device to a similarly equipped MDC 30 or, alternatively, to a mobile telephone transmitter/ receiver for transmission to a receiver connected to a server system. Using the IEEE 522.11x standards for example, data is transmitted directly to a receiver, which may be wired into a network using Ethernet or other network topology. The MDC of the present invention may be capable of wireless communication using either BLUE TOOTH™ or other technologies (such as those described in IEEE 522.11x), and may be used throughout a care giving facility without the disadvantage of requiring cumbersome hardwired devices.

While the medication administration device described above was primarily described in terms of an infusion pump, devices incorporating the principles of the present invention may also be a vital signs monitor or other clinical device interacting with a patient. For example, the medication administration device may also be a patient feeding device.

Furthermore, the institutional communication system 50 as mentioned above numerous times is not meant to be taken in a limited sense. Such a communication system may encompass an entire hospital facility or may be located only in a small area of the hospital. It may also include a communication system in a care-giving facility other than a hospital and may have application to an alternate care facility, such as a patient's home. The above embodiments are described for exemplary purposes only.

In the above detailed description, well-known devices, methods, procedures, and individual components have not been described in detail so as not to obscure aspects of the present invention. Those skilled in the art will understand those devices, methods, procedures, and individual components without further details being provided here. Moreover, while the embodiments disclosed above are described for use in a hospital environment, it will be understood that the system and method may be useful in other environments as well, such as outpatient clinics and other environments where care is delivered to a patient.

While several specific embodiments of the invention have been illustrated and described, it will be apparent that various modifications can be made. Accordingly, it is not intended that the invention be limited, except as by the appended claims.

## Claims

1. A method of reducing a risk of medication errors, the method comprising the steps of:
receiving at a mobile device a first value of an infusion parameter for a medication administration device configured to administer a medication to the patient according to the infusion parameter, the medication administration device being separate from the mobile device;
comparing, at the mobile device, before the medication is administered to the patient by the medication administration device, the first value of the infusion parameter to a hard limit and a soft limit on the infusion parameter,
providing a visual indication at a display screen of the mobile device if the first value of the infusion parameter contravenes the soft limit of the infusion parameter, the indication requiring an user acknowledgement that the first value is to remain in force;
sending a signal, by the mobile device to the medication administration device, to initiate the administration of the medication to the patient by the medication administration device when the first value of the infusion parameter does not contravene a hard limit of the infusion parameter and the user acknowledgement is received when the first value contravenes the soft limit; and
preventing, by the mobile device, the medication administration delivery device from beginning administration of the medication to the patient while the first value of the infusion parameter contravenes the hard limit of the infusion parameter by not sending the signal to the medical administration device.
further comprising:
receiving information associated with the patient and information associated with the medication;
verifying, based on the information associated with the patient and the information associated with the medication, that the right medication is being delivered to the right patient; and
if there is a discrepancy between the information associated with the patient and the information associated with the medication, providing an indication of an alert on a display device associated with the medication administration device,
wherein the medication administration device is configured to receive the first value only after the verifying is complete and there is no discrepancy or the discrepancy has been corrected or overridden by a user.

2. A system comprising a mobile device for reducing a possibility of medication errors, wherein the mobile device comprises a database comprising a hard limit for an infusion parameter and a soft limit for the infusion parameter, and a medication administration device (3) separate from the mobile device comprising a first program loaded onto a computer in the medication administration device (3), which first program is also configured to receive a first value of the infusion parameter for administration of a medication to a patient by the medication administration device, the infusion parameter entered into the medication administration device (3) by an operator and to wirelessly transmit the infusion parameter to the mobile device, wherein the mobile device further comprises a second program loaded onto a computer in the mobile device, which second program is configured to wirelessly receive the infusion parameter from the medication administration device and to retrieve the hard limit and the soft limit from the database, and compare the value of the infusion parameter with the hard limit and the soft limit before the medication is administered to the patient by the medication administration device, the second program further configured to, if the value of the infusion parameter exceeds the soft limit, provide an indication on a display of the mobile device requiring an user acknowledgement that the first value is to remain in force, send a signal to the medication administration device to initiate the administration of the medication to the patient by the medication administration device when the first value of the infusion parameter does not contravene a hard limit of the infusion parameter and the user acknowledgement is received when the first value contravenes the soft limit, and if the value of the infusion parameter exceeds the hard limit, lock out the medication administration device from beginning administration of the medication to the patient by not sending the signal to the medical administration device further comprising a medication administration module configured to:
receive information associated with the patient and information associated with the medication;
verify, based on the information associated with the patient and the information associated with the medication, that the right medication is being delivered to the right patient; and
if there is a discrepancy between the information associated with the patient and the information associated with the medication, provide an indication of an alert on a display device associated with the medication administration device,
wherein the medication administration device is configured to receive the first value only after the verifying is complete and there is no discrepancy or the discrepancy has been corrected or overridden by a user.

3. The system of claim 2 wherein the second program is configured to automatically compare the value of the infusion parameter to the hard limit and the soft limit as the value of the infusion parameter is entered into the medication administration device (3) and immediately provide an alarm if a most recently entered value of the infusion parameter is outside at least one of the soft limit or the hard limit.

4. The system of claim 2, wherein if the first value of the infusion parameter is outside the hard limit, the second program is configured to require a new value of the infusion parameter that is within the hard limit to be entered before operation of the medication administration device can begin.

5. The system of claim 2, further comprising an event logging module configured to:
receive and store information related to entry of a value of an infusion parameter that is outside at least one of the hard limit and the soft limit of a physiological parameter in the database;
analyze the information; and
generate reports related to the information,
wherein the second program is further configured to provide the information to the event logging module.

6. The method of claim 1, further comprising the steps of:
accepting a second value of the infusion parameter;
comparing the second value to the hard limit; and
ceasing prevention of delivery of the medication if the second value of the infusion parameter does not contravene the hard limit.

7. The method of claim 1, further comprising the steps of:
accepting an acknowledgement if the first value of the infusion parameter contravenes the soft limit and is within the hard limit;
preventing delivery of the medication before and until the acknowledgment is accepted if the first value of the infusion parameter contravenes the soft limit and is within the hard limit; and
allowing delivery of the medication after the acknowledgment is accepted if the first value of the infusion parameter contravenes the soft limit and is within the hard limit.

8. The system of any one of claims 2 to 5 further comprising means for consolidating and analyzing information with an event analysis and reporting server.

9. The system of any one of claims 2 to 5 or 8 further comprising means for consolidating and analyzing information adapted to analyze information from more than one medication administration device (3).

10. The system of any one of claims 2 to 5, 8 or 9 further comprising means for consolidating and analyzing information adapted to provide reports concerning an occurrence of events associated with selected areas within an institution, selected treatment protocols, or other categories as identified by the institution to assist the institution in ensuring a proper delivery of medication to patients within the institution.

11. The method of claim 1, further comprising:
retrieving, by the mobile device, from a centralized server, the soft limit and the hard limit.

12. The method of claim 1, wherein the first value is received at the mobile device from the medication administration device.

13. The method of claim 1, wherein the first value is automatically downloaded into the medication administration device from a remote computing device when the verifying is complete.

## Patentansprüche

1. Verfahren zur Verringerung eines Risikos von Fehlern bei der Verabreichung von Arzneimitteln, wobei das Verfahren die folgenden Schritte umfasst:
an einer mobilen Vorrichtung, Empfangen eines ersten Werts eines Infusionsparameters für eine Arzneimittelverabreichungsvorrichtung, die so gestaltet ist, dass sie dem Patienten gemäß dem Infusionsparameter ein Arzneimittel verabreicht, wobei die Arzneimittelverabreichungsvorrichtung von der mobilen Vorrichtung getrennt ist;
bevor das Arzneimittel durch die Arzneimittelverabreichungsvorrichtung dem Patienten verabreicht wird, an der mobilen Vorrichtung Vergleichen des ersten Werts des Infusionsparameters mit einem harten Grenzwert und einem weichen Grenzwert an dem Infusionsparameter;
Bereitstellen einer visuellen Indikation auf einem Bildschirm der mobilen Vorrichtung, wenn der erste Wert des Infusionsparameters gegen weichen Grenzwert des Infusionsparameters verstößt, wobei die Indikation eine benutzerseitige Bestätigung erfordert, dass der erste Wert in Kraft bleiben soll;
Senden eines Signals durch die mobile Vorrichtung an die Arzneimittelverabreichungsvorrichtung, um die Verabreichung des Arzneimittels durch die Arzneimittelverabreichungsvorrichtung an den Patienten einzuleiten, wenn der erste Wert des Infusionsparameters nicht gegen einen harten Grenzwert des Infusionsparameters verstößt und die benutzerseitige Bestätigung empfangen wird, wenn der erste Wert gegen den weichen Grenzwert verstößt; und
durch die mobile Vorrichtung Verhindern, dass die Arzneimittelverabreichungsvorrichtung mit der Verabreichung des Arzneimittels an den Patienten beginnt, während der erste Wert des Infusionsparameters gegen den harten Grenzwert des Infusionsparameters verstößt, indem das Signal nicht an die Arzneimittelverabreichungsvorrichtung gesendet wird;
ferner umfassend:
Empfangen von Informationen, die dem Patienten zugeordnet sind, und von Informationen, die dem Arzneimittel zugeordnet sind;
auf der Basis der Informationen, die dem Patienten zugeordnet sind, und der Informationen, die dem Arzneimittel zugeordnet sind, Verifizieren, dass das richtige Arzneimittel dem richtigen Patienten verabreicht wird; und
wenn zwischen den Informationen, die dem Patienten zugeordnet sind, und den Informationen, die dem Arzneimittel zugeordnet sind, eine Diskrepanz besteht, Bereitstellen einer Indikation einer Warnmeldung auf einer Anzeigevorrichtung, die der Arzneimittelverabreichungsvorrichtung zugeordnet ist;
wobei die Arzneimittelverabreichungsvorrichtung so gestaltet ist, dass sie den ersten Wert ausschließlich nach Abschluss der Verifizierung empfängt, und wenn keine Diskrepanz besteht oder wenn die Diskrepanz korrigiert oder durch einen Benutzer überschrieben worden ist.

2. System, das eine mobile Vorrichtung zur Verringerung eines Risikos von Fehlern bei der Verabreichung von Arzneimitteln umfasst, wobei die mobile Vorrichtung eine Datenbank umfasst, die einen harten Grenzwert für einen Infusionsparameter und einen weichen Grenzwert für den Infusionsparameter umfasst, und eine von der mobilen Vorrichtung getrennte Arzneimittelverabreichungsvorrichtung (3), die ein erstes Programm umfasst, das auf einen Computer in der Arzneimittelverabreichungsvorrichtung (3) geladen ist, wobei das erste Programm so gestaltet ist, dass es einen ersten Wert des Infusionsparameters für die Verabreichung eines Arzneimittels durch die Arzneimittelverabreichungsvorrichtung an den Patienten empfängt, wobei der Infusionsparameter durch eine Bedienungsperson in die Arzneimittelverabreichungsvorrichtung (3) eingegeben wird, und zur drahtlosen Übermittlung des Infusionsparameters an die mobile Vorrichtung;
wobei die mobile Vorrichtung ferner ein zweites Programm umfasst, das auf einen Computer in der mobilen Vorrichtung geladen ist, wobei das zweite Programm so gestaltet ist, dass es den Infusionsparameter drahtlos von der Arzneimittelverabreichungsvorrichtung empfängt und den harten Grenzwert und den weichen Grenzwert aus der Datenbank abruft und den Wert des Infusionsparameters mit dem harten Grenzwert und dem weichen Grenzwert vergleicht, bevor das Arzneimittel durch die Arzneimittelverabreichungsvorrichtung an den Patienten verabreicht wird, wobei das zweite Programm ferner so gestaltet ist, dass es, wenn der Wert des Infusionsparameters den weichen Grenzwert überschreitet, eine Indikation auf einer Anzeige der mobilen Vorrichtung bereitstellt, die eine benutzerseitige Bestätigung erfordert, dass der erste Wert in Kraft bleiben soll, wobei ein Signal an die Arzneimittelverabreichungsvorrichtung gesendet wird, um die Verabreichung des Arzneimittels durch die Arzneimittelverabreichungsvorrichtung an den Patienten einzuleiten, wenn der erste Wert des Infusionsparameters nicht gegen einen harten Grenzwert des Infusionsparameters verstößt und die benutzerseitige Bestätigung empfangen wird, wenn der erste Wert gegen den weichen Grenzwert verstößt, und wobei, wenn der Wert des Infusionsparameters gegen den harten Grenzwert verstößt, die Arzneimittelverabreichungsvorrichtung gegen den Beginn der Verabreichung des Arzneimittels an den Patienten gesperrt wird, indem das Signal nicht an die Arzneimittelverabreichungsvorrichtung gesendet wird;
ferner ein Arzneimittelverabreichungsmodul umfassend, das für folgende Zwecke gestaltet ist:
auf der Basis der Informationen, die dem Patienten zugeordnet sind, und der Informationen, die dem Arzneimittel zugeordnet sind, Verifizieren, dass das richtige Arzneimittel dem richtigen Patienten verabreicht wird; und
wenn zwischen den Informationen, die dem Patienten zugeordnet sind, und den Informationen, die dem Arzneimittel zugeordnet sind, eine Diskrepanz besteht, Bereitstellen einer Indikation einer Warnmeldung auf einer Anzeigevorrichtung, die der Arzneimittelverabreichungsvorrichtung zugeordnet ist;
wobei die Arzneimittelverabreichungsvorrichtung so gestaltet ist, dass sie den ersten Wert ausschließlich nach Abschluss der Verifizierung empfängt, und wenn keine Diskrepanz besteht oder wenn die Diskrepanz korrigiert oder durch einen Benutzer überschrieben worden ist.

3. System nach Anspruch 2, wobei das zweite Programm so gestaltet ist, dass es automatisch den Wert des Infusionsparameters mit dem harten Grenzwert und dem weichen Grenzwert vergleicht, wenn der Wert des Infusionsparameters in die Arzneimittelverabreichungsvorrichtung (3) eingegeben wird, und unverzüglich eine Warnmeldung bereitstellt, wenn ein zuletzt eingegebener Wert des Infusionsparameters gegen wenigstens einen Grenzwert des weichen Grenzwerts oder des harten Grenzwerts verstößt.

4. System nach Anspruch 2, wobei, wenn der erste Wert des Infusionsparameters außerhalb des harten Grenzwerts liegt, das zweite Programm so gestaltet ist, dass ein neuer Wert des Infusionsparameters, der innerhalb des harten Grenzwerts liegt, eingegeben werden muss, bevor der Betrieb der Arzneimittelverabreichungsvorrichtung beginnen kann.

5. System nach Anspruch 2, wobei dieses ferner ein Ereignisprotokollierungsmodul umfasst, das für folgende Zwecke gestaltet ist:
Empfangen und Speichern von Informationen, die sich auf einen Eintrag eines Werts eines Infusionsparameters beziehen, der außerhalb wenigstens des harten Grenzwerts oder des weichen Grenzwerts eines physiologischen Parameters in der Datenbank liegt;
Analysieren der Informationen; und
Erzeugen von Berichten, die sich auf die Informationen beziehen;
wobei das zweite Programm ferner so gestaltet ist, dass es die Informationen an das Ereignisprotokollierungsmodul bereitstellt.

6. Verfahren nach Anspruch 1, wobei dieses ferner die folgenden Schritte umfasst:
Annehmen eines zweiten Werts des Infusionsparameters;
Vergleichen des zweiten Werts mit dem harten Grenzwert; und
Beenden des Verhinderns des Verabreichens des Arzneimittels, wenn der zweite Wert des Infusionsparameters nicht gegen den harten Grenzwert verstößt.

7. Verfahren nach Anspruch 1, wobei dieses ferner die folgenden Schritte umfasst:
Annehmen einer Bestätigung, wenn der erste Wert des Infusionsparameters gegen den weichen Grenzwert verstößt und innerhalb des harten Grenzwerts liegt;
Verhindern der Verabreichung des Arzneimittels vor und bis zu der Annahme der Bestätigung, wenn der erste Wert des Infusionsparameters gegen den weichen Grenzwert verstößt und innerhalb des harten Grenzwerts liegt; und
Zulassen der Verabreichung des Arzneimittels nach Annahme der Bestätigung, wenn der erste Wert des Infusionsparameters gegen den weichen Grenzwert verstößt und innerhalb des harten Grenzwerts liegt.

8. System nach einem der Ansprüche 2 bis 5, wobei dieses ferner ein Mittel zur Konsolidierung und Analyse von Informationen mit einem Server zur Analyse und Berichterstellung in Bezug auf Ereignisse.

9. System nach einem der Ansprüche 2 bis 5 oder 8, wobei dieses ferner Mittel zur Konsolidierung und Analyse von Informationen umfasst, welche Informationen von mehr als einer Arzneimittelverabreichungsvorrichtung analysieren können.

10. System nach einem der Ansprüche 2 bis 5, 8 oder 9, wobei dieses ferner Mittel zur Konsolidierung und Analyse von Informationen umfasst, welche Berichte zu dem Eintreten von Ereignissen bereitstellen, die bestimmten Bereichen innerhalb einer Einrichtung zugeordnet sind, ausgewählten Behandlungsprotokollen oder anderen Bereichen, wie durch die Institution identifiziert, um die Einrichtung dabei zu unterstützen, eine ordnungsgemäße Verabreichung von Arzneimitteln an Patienten in der Einrichtung sicherzustellen.

11. Verfahren nach Anspruch 1, wobei dieses ferner folgendes umfasst:
Abrufen des weichen Grenzwerts und des harten Grenzwerts von einem zentralen Server durch die mobile Vorrichtung.

12. Verfahren nach Anspruch 1, wobei der erste Wert von der Arzneimittelverabreichungsvorrichtung an der mobilen Vorrichtung empfangen wird.

13. Verfahren nach Anspruch 1, wobei der erste Wert von einer entfernten Rechenvorrichtung automatisch in die Arzneimittelverabreichungsvorrichtung heruntergeladen wird, wenn die Verifizierung abgeschlossen ist.

## Revendications

1. Procédé de réduction d'un risque d'erreurs de médication, le procédé comprenant les étapes consistant à :
recevoir sur un dispositif mobile une première valeur d'un paramètre de perfusion pour un dispositif d'administration de médicament conçu pour administrer un médicament au patient selon le paramètre de perfusion, le dispositif d'administration de médicament étant séparé du dispositif mobile ;
comparer, sur le dispositif mobile, avant que le médicament ne soit administré au patient par le dispositif d'administration de médicament, la première valeur du paramètre de perfusion à une limite stricte et à une limite non stricte sur le paramètre de perfusion,
fournir une indication visuelle sur un écran d'affichage du dispositif mobile si la première valeur du paramètre de perfusion transgresse la limite non stricte du paramètre de perfusion, l'indication exigeant que l'utilisateur reconnaisse que la première valeur doit demeurer en vigueur ;
envoyer un signal, par le dispositif mobile au dispositif d'administration de médicament, pour déclencher l'administration du médicament au patient par le dispositif d'administration de médicament lorsque la première valeur du paramètre de perfusion ne transgresse pas une limite stricte du paramètre de perfusion et que l'accusé de réception est reçu lorsque la première valeur transgresse la limite non stricte ; et
empêcher, par le dispositif mobile, le dispositif d'administration de médicament de commencer l'administration du médicament au patient lorsque la première valeur du paramètre de perfusion transgresse la limite stricte du paramètre de perfusion en n'envoyant pas le signal au dispositif d'administration médical,
comprenant en outre les étapes consistant à :
recevoir des informations associées au patient et des informations associées au médicament ;
vérifier, sur la base des informations associées au patient et des informations associées au médicament, que le bon médicament est administré au bon patient ; et
en cas de divergence entre les informations associées au patient et les informations associées au médicament, fournir une indication d'une alerte sur un dispositif d'affichage associé au dispositif d'administration de médicament,
le dispositif d'administration de médicament étant conçu pour recevoir la première valeur seulement après que la vérification est terminée et qu'il n'y a aucune anomalie ou que l'anomalie a été corrigée ou remplacée par un utilisateur.

2. Système comprenant un dispositif mobile pour réduire une possibilité d'erreurs de médication, le dispositif mobile comprenant une base de données comprenant une limite stricte pour un paramètre de perfusion et une limite non stricte pour le paramètre de perfusion, et un dispositif d'administration de médicament (3) séparé du dispositif mobile comprenant un premier programme chargé sur un ordinateur dans le dispositif d'administration de médicament (3), ledit premier programme étant également conçu pour recevoir une première valeur du paramètre de perfusion pour administrer un médicament à un patient par le dispositif d'administration de médicament, le paramètre de perfusion étant introduit dans le dispositif d'administration de médicament (3) par un opérateur et pour transmettre le paramètre de perfusion au dispositif mobile sans fil,
le dispositif mobile comprenant en outre un second programme chargé sur un ordinateur dans le dispositif mobile, ledit second programme étant conçu pour recevoir sans fil le paramètre de perfusion du dispositif d'administration de médicament et pour récupérer la limite stricte et la limite non stricte de la base de données, et pour comparer la valeur du paramètre de perfusion avec la limite stricte et la limite non stricte avant que le médicament soit administré au patient par le dispositif d'administration de médicament, le second programme étant de plus conçu pour, si la valeur du paramètre de perfusion dépasse la limite non stricte, fournir une indication sur un affichage du dispositif mobile exigeant un accusé de réception par l'utilisateur que la première valeur doit être maintenue en vigueur, envoyer un signal au dispositif d'administration de médicament pour qu'il déclenche l'administration du médicament au patient par le dispositif d'administration de médicament lorsque la première valeur du paramètre de perfusion ne transgresse pas une limite stricte du paramètre de perfusion et que l'accusé de réception est reçu lorsque la première valeur transgresse la limite non stricte, et si la valeur du paramètre de perfusion dépasse la limite stricte, empêcher le dispositif d'administration de médicament de commencer l'administration du médicament au patient en n'envoyant pas le signal au dispositif d'administration de médicament,
comprenant en outre un module d'administration de médicament conçu pour :
recevoir les informations associées au patient et les informations associées au médicament ;
vérifier, sur la base des informations associées au patient et des informations associées au médicament, que le bon médicament est administré au bon patient ; et
en cas de divergence entre les informations associées au patient et les informations associées au médicament, fournir une indication d'une alerte sur un dispositif d'affichage associé au dispositif d'administration de médicament,
le dispositif d'administration de médicament étant conçu pour recevoir la première valeur seulement après que la vérification est terminée et qu'il n'y a aucune anomalie ou que l'anomalie a été corrigée ou remplacée par un utilisateur.

3. Système selon la revendication 2, le second programme étant conçu pour comparer automatiquement la valeur du paramètre de perfusion à la limite stricte et à la limite non stricte lorsque la valeur du paramètre de perfusion est entrée dans le dispositif d'administration de médicament (3) et pour émettre immédiatement une alarme si une valeur entrée en dernier du paramètre de perfusion se situe en dehors de la limite non stricte et/ou de la limite stricte.

4. Système selon la revendication 2, si la première valeur du paramètre de perfusion se situe en dehors de la limite stricte, le second programme étant conçu pour exiger qu'une nouvelle valeur du paramètre de perfusion qui se situe dans la limite stricte soit entrée avant que l'opération du dispositif d'administration de médicament puisse commencer.

5. Système selon la revendication 2, comprenant en outre un module d'enregistrement d'événements conçu pour :
recevoir et stocker les informations relatives à l'entrée d'une valeur d'un paramètre de perfusion qui se situe à l'extérieur de la limite stricte et/ou de la limite non stricte d'un paramètre physiologique dans la base de données ;
analyser les informations ; et
générer des rapports relatifs aux informations,
le second programme étant en outre conçu pour fournir les informations au module d'enregistrement d'événements.

6. Procédé selon la revendication 1, comprenant en outre les étapes consistant à :
accepter une seconde valeur du paramètre de perfusion ;
comparer la seconde valeur à la limite stricte ; et
cesser l'empêchement de l'administration du médicament si la seconde valeur du paramètre de perfusion ne transgresse pas la limite stricte.

7. Procédé selon la revendication 1, comprenant en outre les étapes consistant à :
accepter un accusé de réception si la première valeur du paramètre de perfusion transgresse la limite non stricte et se situe à l'intérieur de la limite stricte ;
empêcher l'administration du médicament avant et jusqu'à ce que l'accusé de réception soit accepté si la première valeur du paramètre de perfusion transgresse la limite non stricte et se situe dans la limite stricte ; et
permettre l'administration du médicament après que l'accusé de réception est accepté si la première valeur du paramètre de perfusion transgresse la limite non stricte et se situe à l'intérieur de la limite stricte.

8. Système selon l'une quelconque des revendications 2 à 5 comprenant en outre un moyen pour consolider et analyser des informations avec un serveur d'analyse et de rapport d'événements.

9. Système selon l'une quelconque des revendications 2 à 5 comprenant en outre un moyen pour consolider et analyser des informations conçu pour analyser des informations provenant de plus d'un dispositif d'administration de médicament (3).

10. Système selon l'une quelconque des revendications 2 à 5 comprenant en outre un moyen pour consolider et analyser les informations conçues pour fournir des rapports concernant la survenue d'événements associés à des zones sélectionnées dans un établissement, des protocoles de traitement sélectionnés ou d'autres catégories identifiées par l'établissement pour aider l'établissement à assurer une bonne livraison des médicaments aux patients dans l'établissement.

11. Procédé selon la revendication 1, comprenant en outre l'étape consistant à :
récupérer, par le dispositif mobile, à partir d'un serveur centralisé, la limite non stricte et la limite stricte.

12. Procédé selon la revendication 1, la première valeur étant reçue sur le dispositif mobile en provenance du dispositif d'administration de médicament.

13. Procédé selon la revendication 1, la première valeur étant automatiquement téléchargée dans le dispositif d'administration de médicament à partir d'un dispositif informatique distant lorsque la vérification est terminée.
